# EUROPEAN PATENT SPECIFICATION

(11) **EP 1 605 565 B1**
(45) Date of publication and mention of the grant of the patent: **17.10.2007**
(21) Application number: 05290978.5
(22) Date of filing: 04.05.2005
(51) Int. Cl.: H01T 23/00

(54) **Negative ion generator using carbon fiber**
Negative Ionengenerator mit Kohlenstoff-Faser
Générateur d'ions négatifs utilisant des fibres de carbon

(30) Priority: 18.05.2004 KR 2004035348
(43) Date of publication of application: 14.12.2005
(73) Proprietor: LG ELECTRONICS INC., Seoul (KR)
(72) Inventor: Kim, Hwa-Nyeon, Bucheon Gyeonggi-Do (KR); Hong, Hyung-Ki, Dongan-Gu Anyang Gyeonggi-Do (KR); Jung, Min-Jae, Seocho-Gu Seoul (KR); Ji, Kwang-Sun, 2-Dong Seongdong-Gu Seoul (KR); Lee, Don-Hee, Dongan-gu, Anyang, Gyeonggi-do (KR); Park, Sang-Ho, Chanwon Gyeongsangnam-Do (KR); Kim, Young-Woo, Jangyu-Myeon Gimhae Gyeongsangnam-Do (KR); Kang, Byeong-Gyu, Gimhae Gyeongsangnam-Do (KR)
(74) Representative: Loisel, Bertrand

(56) References cited:
- EP-A- 0 048 102
- DE-A1- 19 931 662

## Description

### BACKGROUND OF THE INVENTION

### 1. Field of the Invention

The present invention relates to a negative ion generator, and particularly, to a negative ion generator using a carbon fiber capable of facilitating its manufacturing process, improving negative ion generation efficiency and implementing improved antimicrobial and sterilizing functions by distributing metallic particles in an activated carbon fiber and then applying a voltage thereto.

### 2. Description of the Background Art

As problems caused by air pollution such as smoke, sand dust phenomena or the like have got worse recently, people are being increasingly interested in clean and fresh air. Since the number of people suffering from various kinds of respiratory diseases or having allergies to polluted air is increasing, several attempts to purify the polluted air by generating negative ions are being made in different ways over various fields.

The negative ion means a state that a molecule such as oxygen or nitrogen in the air has negative charge. It has been reported that such a negative ion is very good for a human body and can effectively remove dust and odor. For this reason, an ion generator is provided in an appliances such as an air purifier, a water purifier, a hair dryer or the like. In contrast, a state that a molecule has positive charge is called a positive ion, which is known to be harmful to a human body because it causes vomiting or dizziness. However, since the positive ion can carry out a sterilizing operation in connection with the negative ion, an apparatus that can generate positive and negative ions at the same time is being released.

The ion generator is varying in function. Besides generating negative ions which is good for the human body, functions of killing germs in the air and filtering fine dust are being developed.

However, the ion generator in accordance with the conventional art has problems in that negative ion generation efficiency is low. Also, a system using an ion generator is provided with a filter and the like, which are separate components, to additionally perform an air purifying function. For this reason, the configuration of the system becomes complicated, which results in the difficult manufacture and management and a cost increase.

An ion generator is known from DE-A-19931662, having electrodes consisting of conductive fibers.

### SUMMARY OF THE INVENTION

Therefore, an object of the present invention is to provide a negative ion generator using an inexpensive carbon fiber, improving negative ion generation efficiency, and easily manufactured while providing both antimicrobial and sterilizing functions.

To achieve these and other advantages and in accordance with the purpose of the present invention, as embodied and broadly described herein, there is provided a negative ion generator using a carbon fiber comprising: a carbon fiber in which metallic particles are distributed; an electrode connected to the carbon fiber and applying a voltage thereto; and a power unit for supplying power to the electrode.

The foregoing and other objects, features, aspects and advantages of the present invention will become more apparent from the following detailed description of the present invention when taken in conjunction with the accompanying drawings.

### BRIEF DESCRIPTION OF THE DRAWINGS

The accompanying drawings, which are included to provide a further understanding of the invention and are incorporated in and constitute a unit of this specification, illustrate embodiments of the invention and together with the description serve to explain the principles of the invention.

In the drawings:
Figure 1 is a sectional view showing a negative ion generator using a carbon fiber in accordance with a first embodiment of the present invention;
Figure 2 is an enlarged picture taken before Ag is distributed in a felt-type activated carbon fiber;
Figure 3 is an enlarged picture taken after Ag is distributed in the felt-type activated carbon fiber;
Figure 4 is a sectional view of a forced-convention air purifier employing the first embodiment of the present invention;
Figure 5 is a sectional view showing a negative generator using a carbon fiber in accordance with a second embodiment of the present invention;
Figure 6 is a graph illustrating the amount of negative ions generated in accordance with the first and second embodiments, which is measured in a space of 1.0m³ at a distance of 30cm from the negative ion generator;
Figure 7 is a graph illustrating the amount of negative ions generated in accordance with the first embodiment, which is measured in a space of 1.0m³ at a distance of 50cm from the negative ion generator; and
Figure 8 is a graph illustrating the amount of negative ions generated in accordance with the second embodiment, which is measured in a space of 1.0m³ at a distance of 50cm from the negative ion generator.

### DETAILED DESCRIPTION OF THE PREFERRED EMBODIMENTS

Reference will now be made in detail to the preferred embodiments of the present invention, examples of which are illustrated in the accompanying drawings.

A plurality of embodiments in accordance with the present invention may exist, and the most preferred embodiment will now be described.

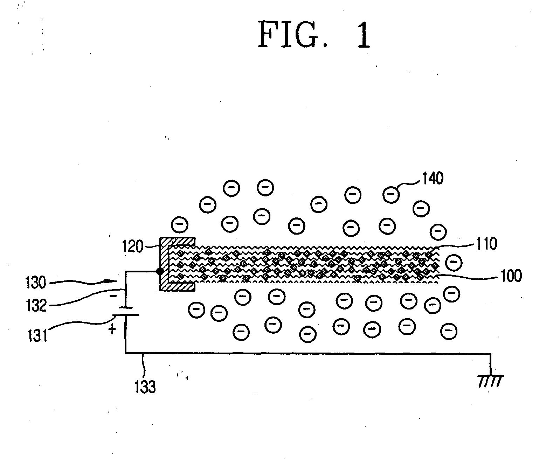

Figure 1 is a sectional view showing a negative ion generator using a carbon fiber in accordance with a first embodiment of the present invention.

As shown, the negative ion generator in accordance with the present invention includes: an activated carbon fiber 100 in which metallic particles 110 are distributed; an electrode 120 connected to the activated carbon fiber 100 and applying a voltage thereto; and a power unit 130 for supplying power to the electrode 120.

Ag, Pt, Au, Cu, Al, Cr, W, Mo or the like may be used as the metallic particle 110, and the metallic particles 110 are evenly distributed in the activated carbon fiber 100. The activated carbon fiber 100 will be described afterwards.

In the present embodiment, Ag is used as the metallic particle 110. It is a known fact that said Ag can kill germs. Accordingly, said Ag is used to add an antimicrobial function to the negative ion generator.

Preferably, the electrode is attached to a side of the activated carbon fiber 100. When a voltage is applied to the activated carbon fiber 100 through the electrode 120, the activated carbon fiber can be used even as a heating source of a heater because of its high electric conductivity. Also, the activated carbon fiber 100 can burn and remove pollutants attached thereto as the electrode applies a high voltage thereto.

The power unit 130 includes: a battery 131 for supplying a DC voltage; a first line 132 connecting one side of the battery 131 with the electrode 120; and a second line 133 for grounding the other side of the battery 131.

The battery 131 may supply a DC voltage after changing an AC voltage supplied to homes to a DC voltage. In such case, the battery 131 may include a converter for converting the AC voltage into the DC voltage.

Carbon constituting the activated carbon fiber 100 is a material used for an absorption filter for purifying indoor air or water. Since said carbon has a surface area of larger than 100 m²/g, it can purify the air by absorbing harmful materials.

Preferably, the activated carbon fiber 100 is made by producing a carbon yarn in a felt type. The felt-type activated carbon fiber 100 can easily implement desired size and shape, and can easily hold and confine the metallic particle by being entangled. Undescribed reference numeral 140 in Figure 1 is a generated negative ion.

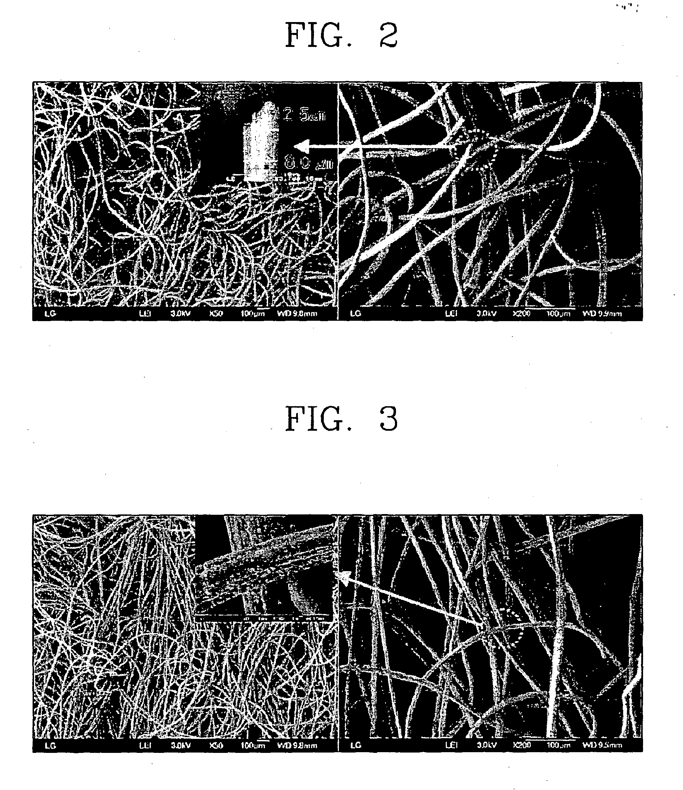

A structure of the activated carbon fiber 100 will now be described in more detail with reference to Figures 2 and 3. Figure 2 is an enlarged picture taken before Ag is distributed in the felt-type activated carbon yarn.

As shown, the activated carbon fiber has a thickness of about a few µm, and is complicatedly entangled. The activated carbon fiber is not connected but is cut into several sections. Thus, each end of the section has a cut surface. The cut surface is not smooth but rough and has a tip. These can be checked by the enlarged picture of Figure 2.

A negative ion is generated from the tip. Namely, all of very fine carbon fiber ends beyond count are used as tips from which negative ions are generated, so that a large amount of negative ions can be stably generated.

Figure 3 is an enlarged picture taken after Ag is distributed in the felt-type activated carbon fiber. As shown, Ag is evenly distributed in the activated carbon fiber.

The operation of the present invention will now be described.

After metallic particles such as Ag are evenly distributed in the activated carbon fiber, a negative (-) voltage is applied to the activated carbon fiber. Then, a large amount of negative ions are generated from the entire surface of the activated carbon fiber. Also, the polluted air passing through the activated carbon fiber is absorbed by the activated carbon fiber so as to be purified, and germs contained in the air are killed by Ag particles. Also, since high temperature heat is generated at the end of the carbon fiber, where the negative ion is generated, sterilization can be carried out by the high temperature heat.

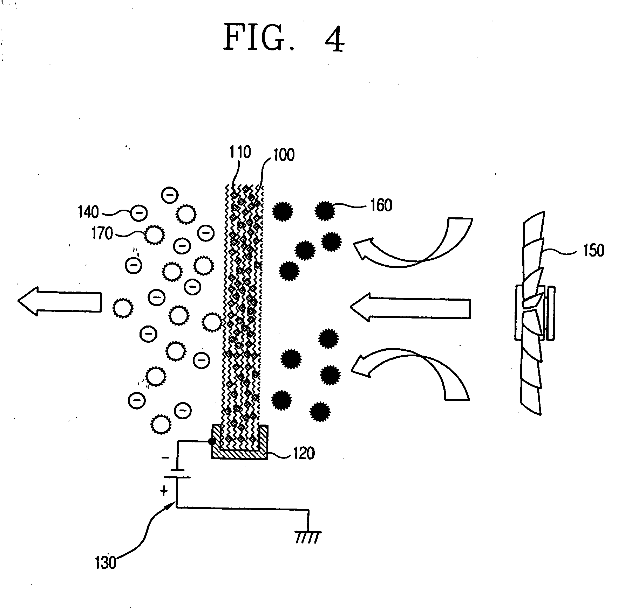

Figure 4 is a sectional view of a forced-convention air purifier employing the first embodiment of the present invention.

As shown, when the polluted indoor air 160 is introduced toward an activated carbon fiber 100 through a fan 150, the activated carbon fiber 10 purifies the air by its large surface area, kills germs, and emits negative ions. Namely, the activated carbon fiber absorbs harmful gases or impurities, and the distributed metallic particles 110 such as Ag kill germs or bacteria. Then, the air 170 purified in such a manner is emitted together with negative ions 140. At this time, since the high temperature heat is generated at the end portion of the activated carbon fiber where the negative ion 140 is generated, an additional sterilization effect can be obtained as mentioned above.

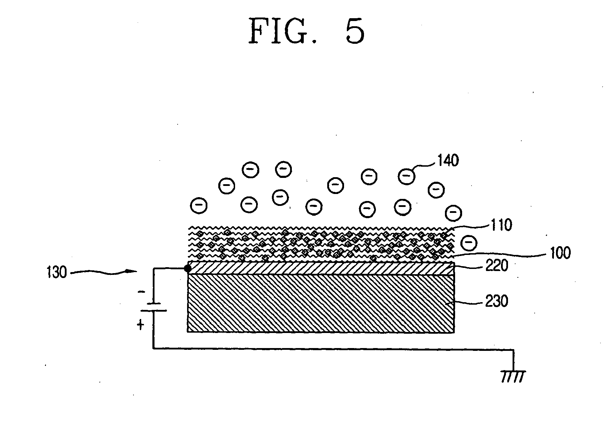

Figure 5 is a sectional view showing a negative ion generator using a carbon fiber in accordance with a second embodiment of the present invention.

In the second embodiment of the present invention, a structure of an electrode is different from that of the first embodiment. As shown in Figure 5, an entire surface of one side of the electrode 220 is in contact with the activated carbon fiber 100. Also, an entire surface of the other electrode 220 is in contact with a non-conductive substrate 230.

In the second embodiment, a voltage applied from a power unit 130 is evenly applied to the activated carbon fiber 100, so that negative ions 140 are uniformly generated. Namely, the negative ion can be uniformly generated regardless of time. However, such uniform negative ion generation interrupts communication between the air and the activated carbon fiber 100. For this reason, the negative ion generator in accordance with the second embodiment is not appropriate to absorb foreign substances or remove odors.

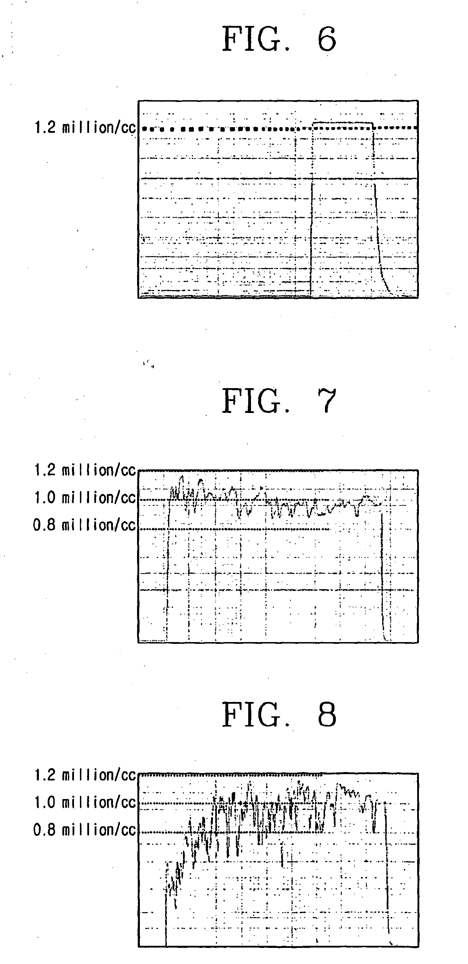

Effects of the present invention will now be described with reference to Figures 6, 7, and 8.

Figure 6 is a graph illustrating the amount of negative ions generated in accordance with the first and second embodiments, which is measured in a space of 1.0m³ at a distance of 30cm from the -negative ion generator, and Figure 7 is a graph illustrating the amount of negative ions generated in accordance with the first embodiment, which is measured in a space of 1.0m³ at a distance of 50cm from the negative ion generator. Figure 8 is a graph illustrating the amount of negative ions generated in accordance with the second embodiment, which is measured in a space of 1.0m³ at a distance of 50cm from the negative ion generator.

In general, it is determined that a negative ion generator is usable when the amount of negative ions, which is measured in a space of 1.0m³ at a distance of 30cm from the negative ion generator, is more than 1.0 million/cc.

In Figure 6, the amount of negative ions exceeds 1.2 million/cc, and in Figure 7, the ion generation is unstable at the initial stage but gets stable over time as the amount of negative ions generated exceeds 1.0 million/cc. In Figure 8, negative ion generation is stable as compared to that shown in Figure 7.

Accordingly, in the present invention, negative ion generation efficiency is improved compared to the conventional art, and a sterilization effect and a filtering function can be also provided. Also, an activated carbon fiber can be molded arbitrarily, so that the negative ion generator can be manufactured more easily.

As the present invention may be embodied in several forms without departing from the essential characteristics thereof, it should also be understood that the above-described embodiments are not limited by any of the details of the foregoing description, unless otherwise specified, but rather should be construed broadly within its scope as defined in the appended claims, and therefore all changes and modifications that fall within the metes and bounds of the claims, or equivalence of such metes and bounds are therefore intended to be embraced by the appended claims.

## Claims

1. A negative ion generator comprising:
an activated carbon fiber (100) in which metallic particles (110) are distributed;
an electrode (120) connected to the activated carbon fiber (100) and applying a voltage thereto; and
a power unit (130) for supplying power to the electrode (120).

2. The negative ion generator of claim 1, wherein the activated carbon fiber (100) is manufactured in a felt type.

3. The negative ion generator of claim 1, wherein the metallic particle (110) is one of Ag, Pt, Au, Cu, Al, Cr, W, and Mo.

4. The negative ion generator of claim 1, wherein the electrode (120) is attached to a side of the activated carbon fiber (100).

5. The negative ion generator of claim 1, wherein an entire surface of one side of the electrode (220) is in contact with the activated carbon fiber (100).

6. The negative ion generator of claim 5, wherein
a non-conductive substrate (230) is in contact with an entire surface of the other side of the electrode (220).

7. The negative ion generator of claim 1, wherein the power unit comprises:
a battery (131) for supplying a DC power;
a first line (132) for connecting one side of the battery with the electrode; and
a second line (133) grounding the other side of the battery.

8. The negative ion generator of claim 7, wherein the power unit (130) further comprises:
a converter for converting an alternating current (AC) to a direct current (DC).

## Patentansprüche

1. Generator negativer Ionen, umfassend:
eine Aktivkohlefaser (100), in welcher metallische Partikel (110) verteilt sind;
eine Elektrode (120), die mit der Aktivkohlefaser (100) verbunden ist, und Anlegen einer Spannung an diese, und
eine Stromversorgungseinheit (130) Versorgung der Elektrode (120) mit Strom.

2. Generator negativer Ionen gemäß Anspruch 1, wobei die Aktivkohlefaser (100) in einem Filz-Typ hergestellt wird.

3. Generator negativer Ionen gemäß Anspruch1, wobei das metallische Partikel (110) eines aus Ag, Pt, Au, Cu, Al, Cr, W und Mo ist.

4. Generator negativer Ionen gemäß Anspruch 1, wobei die Elektrode (120) an einer Seite der Aktivkohlefaser (100) angebracht ist.

5. Generator negativer Ionen gemäß Anspruch 1, wobei die gesamte Oberfläche einer Seite der Elektrode (220) sich im Kontakt mit der Aktivkohlefaser (100) befindet.

6. Generator negativer Ionen gemäß Anspruch 5, wobei
ein nicht-leitfähiges Substrat (230) sich im Kontakt mit der gesamten Oberfläche der anderen Seite der Elektrode (220) befindet.

7. Generator negativer Ionen gemäß Anspruch 1, wobei die Stromerzeugungseinheit Folgendes umfasst:
eine Batterie (131) zur Bereitstellung von Gleichstrom (DC);
eine erste Leitung (132) zur Verbindung einer Seite der Batterie mit der Elektrode; und
eine zweite Leitung (133), die die andere Seite der Batterie erdet.

8. Generator negativer Ionen gemäß Anspruch 7, wobei die Stromerzeugungseinheit (130) ferner Folgendes umfasst:
einen Wandler zum Umwandeln eines Wechselstroms (AC) zu einem Gleichstrom (DC).

## Revendications

1. Un générateur d'Ions négatifs comprenant :
des fibres de charbon actif (100) dans lesquelles des particules métalliques (110) sont réparties:
une électrode (120) connectée aux fibres de charbon actif (100) et leur appliquant une tension; et
une unité d'alimentation (130) pour fournir de l'énergie à l'électrode (120).

2. Le générateur d'Ions négatifs de la revendication 1, dans lequel les fibres de charbon actif (100) sont fabriquées sous une forme de feutre.

3. Le générateur d'ions négatifs de la revendication 1, dans lequel les particules métalliques (110) consistent en un des métaux suivants : Ag, Pt, Au, Cu, Al, Cr, W et Mo.

4. Le générateur d'ions négatifs de la revendication 1, dans lequel l'électrode (120) est fixée à un côté des fibres de charbon actif (100).

5. Le générateur d'lons négatifs de la revendication 1, dans lequel une surface entière d'une face de l'électrode (220) est en contact avec les fibres de charbon actif (100).

6. Le générateur d'ions négatifs de la revendication 5, dans lequel
un substrat non conducteur (230) est en contact avec une surface entière de l'autre face de l'électrode (220)

7. Le générateur d'ions négatifs de la revendication 1, dans lequel l'unité d'alimentation comprend :
une batterie (131) pour fournir de l'énergie à courant continu;
une première ligne (132) pour connecter un côté de la batterie à l'électrode; et
une deuxième ligne (133) mettant à la masse l'autre côté de la batterie.

8. Le générateur d'lons négatifs de la revendication 7, dans lequel l'unité d'alimentation (130) comprend en outre :
un convertisseur pour convertir un courant alternatif (AC) en un courant continu (DC).
